Europäisches Patentamt

European Patent Office

Office européen des brevets

(19)

(11) **EP 1 522 850 A2**

(12) **DEMANDE DE BREVET EUROPEEN**

(43) Date de publication:
**13.04.2005 Bulletin 2005/15**

(51) Int Cl.[7]: **G01N 33/24**, G01N 27/02, G01R 27/02

(21) Numéro de dépôt: **04292263.3**

(22) Date de dépôt: **21.09.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **10.10.2003 FR 0311958**

(71) Demandeur: **Institut Français du Pétrole**
**92852 Rueil Malmaison Cédex (FR)**

(72) Inventeur: **Fleury, Marc**
**78170 La Celle-Saint-Cloud (FR)**

(54) **Méthode et dispositif pour mesurer l'anisotropie de résistivité d'échantillons de roche présentant des litages**

(57) **-**Méthode et dispositif pour mesurer l'anisotropie de résistivité de roches présentant des litages de conductivité différentes, tels que des laminations.

**-** - La méthode comporte la mise en place de l'échantillon saturé par un premier fluide, dans un dispositif de mesures pétrophysiques permettant de le soumettre à des opérations de drainage par injection sous pression d'un deuxième fluide. Des couples d'électrodes $(E_2, E'_1, E'_2)$ sont plaquées contre sa paroi périphérique permettant l'application d'un courant électrique (I) et la détection des différences de potentiel $(\Delta U)$ apparaissant entre des points d'application distincts en réponse à l'application du courant électrique. Durant ces opérations, on établit au moins un palier de pression d'injection du deuxième fluide et on réalise de mesures précises en continu des variations de l'impédance électrique complexe de l'échantillon à plusieurs fréquences durant une phase de déplacement du fluide saturant. Dans un premier temps, on réalise des mesures de l'impédance présentée par l'échantillon dans une position où les litages sont orientées sensiblement transversalement au champ électrique (E) créé par l'application du courant électrique et dans un deuxième temps, on réalise les mêmes mesures dans une autre position où les litages sont orientées sensiblement dans la direction du champ électrique. On détermine alors l'anisotropie de résistivité.

**-** Applications par exemple aux mesures pétrophysiques sur échantillons de roche poreux.

**FIG.3A**

$$R = \Delta U/I$$

## Description

La présente invention a pour objet une méthode et un dispositif pour mesurer l'anisotropie de résistivité de roches présentant des litages, tels que des laminations. Ce litage est du à l'existence de couches argileuses ou couches de sables compactés d'une granulométrie différentes. Lorsque la formation est saturée à 100 % d'eau, le contraste de résistivité est faible (facteur 3 par exemple). Mais dans les zones à huiles, ce contraste devient très important à cause d'une saturation en eau très différente.

La mesure de l'indice de résistivité de carottes présentant de telles litages, est nécessaire pour obtenir une estimation précise de la saturation en eau à partir de données de diagraphie obtenues par exemple par la technique de mesure pendant forage (MWD).

La connaissance de l'anisotropie de résistivité dans deux directions privilégiées est utile pour déterminer la saturation en eau du milieu poreux se trouvant entre les laminations. En effet, les outils diagraphiques de puits ont souvent une résolution verticale insuffisante pour détecter les fluctuations de résistivité résultant de l'accumulation des différents litages en fonction de la profondeur. De plus, les laminations contiennent peu d'hydrocarbures et peuvent être très peu perméables car ils sont souvent composés essentiellement d'argile. Ainsi, il est connu que la mesure de la résistivité moyenne dans une direction ne permet pas de déduire la saturation en eau dans les couches pouvant produire les hydrocarbures.

## Etat de la technique

Par les brevets EP 701.128 (US 5.610.524) et FR 2.581.573 (US 5 979 223) du demandeur, notamment on connaît différentes méthodes et des dispositifs de mesure en continu de la courbe de l'indice de résistivité d'un échantillon solide initialement saturé par un premier fluide mouillant, tel qu'un échantillon géologique, indépendamment de la courbe de pression capillaire. L'échantillon solide poreux est contenue dans une gaine étanche qui est placée dans une cellule de confinement allongée entre deux embouts. Des canaux au travers des deux embouts communiquent avec un système d'injection permettant d'injecter un deuxième fluide non mouillant dans l'échantillon à une première extrémité de la cellule et de drainer le premier fluide hors de la cellule à l'extrémité opposée, au travers d'une membrane semi-perméable, perméable au premier fluide. L'échantillon est contenu dans une gaine et soumis à une pression radiale par injection d'huile sous pression dans l'espace annulaire entre le corps de la cellule et la gaine. Une membrane mouillable seulement par le deuxième fluide est interposée entre l'échantillon et la première extrémité de la cellule pour réaliser des opérations de réimbibition.

Des électrodes interposées entre l'échantillon et sa gaine permettent l'application d'un courant électrique et la détection des différences de potentiel apparaissant entre des points distincts en réponse à l'application du courant électrique. Les électrodes étant connectées à un appareil de mesure de l'impédance complexe de l'échantillon. L'extension longitudinale des électrodes est relativement importante rapportée à la longueur de l'échantillon de façon à impliquer la plus grande partie possible du volume de l'échantillon dans les mesures d'impédance tout en évitant les courts-circuits par les extrémités de l'échantillon susceptibles de fausser les mesures.

On applique un ou plusieurs paliers de pression d'injection, on mesure les variations continues de l'indice de résistivité en fonction de la variation de saturation moyenne sans attendre que s'établisse les équilibres capillaires.

L'espace annulaire entre la gaine et la paroi extérieure de la cellule étant sous pression élevée, les conducteurs électriques reliant les électrodes à l'appareil de mesure traversent la paroi extérieure de la cellule par des traversées étanches (connecteurs à perles de verre par exemple).

Pour améliorer la précision des mesures quand on opère à des fréquences beaucoup plus élevées comprises par exemple dans la plage 100 kHz - 10MHz, on peut utiliser avantageusement le dispositif de connexion décrit dans le brevet FR 2.809.821 (US 6.571.606) du demandeur qui permet de réaliser la connexion par câble blindé des électrodes à un appareil de mesure, situés de part et d'autre d'une paroi séparant une enceinte sous pression du milieu extérieur.

## La méthode et le dispositif selon l'invention

La méthode selon l'invention permet de mesurer l'anisotropie de résistivité d'un échantillon poreux traversé par au moins une litage de conductivité différente, tel qu'une lamination, cet échantillon étant initialement saturé par un premier fluide. Elle comporte la mise en place de l'échantillon dans un dispositif comprenant une cellule de confinement allongée, avec un premier filtre semi-perméable, perméable au premier fluide et disposé sensiblement au contact d'une première extrémité de l'échantillon, et des moyens pour l'injection sous pression d'un deuxième fluide au travers d'une deuxième extrémité de l'échantillon, l'application contre l'échantillon d'électrodes permettant l'application d'un courant électrique et la détection des différences de potentiel apparaissant entre des points d'application distincts en réponse à l'application du courant électrique, l'établissement d'au moins un palier de pression d'injection du deuxième fluide et la réalisation de mesures précises en continu des variations de l'impédance électrique complexe de l'échantillon à plusieurs fréquences durant une phase de déplacement du fluide saturant.

La méthode se distingue en ce que :

- a) on réalise des mesures de l'impédance présentée par l'échantillon dans une position où le ou les litage(s) sont orientées sensiblement transversalement au champ électrique créé par l'application du courant électrique ;

- b) on réalise des mesures de l'impédance présentée par l'échantillon dans une position où les litages sont orientées sensiblement dans le sens du champ électrique créé par l'application du courant électrique ; et

- c) on détermine l'anisotropie de résistivité.

Suivant un premier mode d'implémentation, on change l'orientation de l'échantillon par rapport à la direction fixe du champ électrique créé par l'application du courant électrique, avant de procéder à l'étape b), de façon que le litage soit sensiblement dans la même direction que le champ électrique.

Suivant un autre mode d'implémentation, on change la direction du champ électrique créé par l'application du courant électrique relativement à l'échantillon, avant de procéder à l'étape b).

On peut utiliser des électrodes dont la longueur est comprise entre ¼ et ¾ de la longueur de l'échantillon et par exemple de l'ordre de la moitié de la longueur de l'échantillon.

Suivant un mode de mise en oeuvre, on réalise des mesures précises en continu des variations de l'impédance électrique complexe de l'échantillon à plusieurs fréquences durant une phase de drainage.

Suivant un mode de mise en oeuvre, on réalise des mesures précises en continu des variations de l'impédance électrique complexe de l'échantillon à plusieurs fréquences durant une phase d'imbibition.

Le dispositif selon l'invention permet de mesurer l'anisotropie de résistivité d'un échantillon poreux traversé par au moins un litage de conductivité différente, tel qu'une lamination, comprenant une cellule de confinement pour un échantillon initialement saturé par un premier fluide, des paires d'électrodes plaquées contre la périphérie de l'échantillon permettant l'application d'un courant électrique et la détection des différences de potentiel apparaissant entre des points distincts de l'échantillon en réponse à l'application du courant électrique, les électrodes étant connectées à un appareil de mesure de l'impédance de l'échantillon, un premier filtre semi-perméable, perméable au premier fluide et disposé sensiblement au contact d'une première extrémité de l'échantillon, et des moyens d'injection pour l'injection sous pression d'un deuxième fluide au travers d'une deuxième extrémité de l'échantillon.

Il est caractérisé en ce qu'il comporte une pluralité de couples d'électrodes répartis sur le pourtour de l'échantillon qui peuvent être sélectivement connectées à l'appareil de mesure de façon à orienter le champ électrique créé par application de courant électrique à l'échantillon respectivement dans la même direction que le litage, à l'étape b) sensiblement et dans une direction transverse à cette couche, à l'étape a).

Présentation des figures

D'autres caractéristiques et avantages apparaîtront à la lecture de la description ci-après d'un exemple non limitatif de réalisation, en se référant aux dessins annexés où :

- la figure 1 montre schématiquement en coupe longitudinale une cellule de mesure permettant la mesure de la résistivité d'un échantillon poreux ;

- la figure 2 montre en coupe transversale la disposition des électrodes autour d'un échantillon permettant d'y injecter un courant électrique et de détecter la différence de potentiel engendrée par le passage du courant au travers de l'échantillon ;

- les figures 3A, 3B montrent une coupe transversale de l'échantillon dans sa gaine de confinement, disposé de façon que le litage soit sensiblement perpendiculaire à la direction du champ électrique qui s'établit entre les électrodes, en réponse à l'application du courant électrique ;

- les figures 4A, 4B montrent une coupe transversale du même échantillon dans sa gaine de confinement, disposé de façon que le litage soit sensiblement parallèle à la direction du champ électrique ; et

- les figures 5A, 5B montrent un agencement de plusieurs couples d'électrodes connectables sélectivement à l'appareil de mesure d'impédance, qui permettent de modifier l'orientation relative du litage par rapport à la direction du champ électrique traversant effectivement l'échantillon et ainsi d'éviter les manipulations de celui-ci entre les étapes de mesure.

## DESCRIPTION DETAILLEE

La méthode selon l'invention peut être mise en oeuvre par un système expérimental destiné à la mesure des variations de l'indice de résistivité d'un échantillon solide poreux, consécutifs à des déplacements forcés d'un premier fluide mouillant conducteur de l'électricité tel que de la saumure par exemple, par injection d'un deuxième fluide non conducteur tel que de l'huile par exemple, (phase de drainage) ou du deuxième fluide par le premier (phase d'imbibition) tel que celui décrit dans les brevets précités du demandeur.

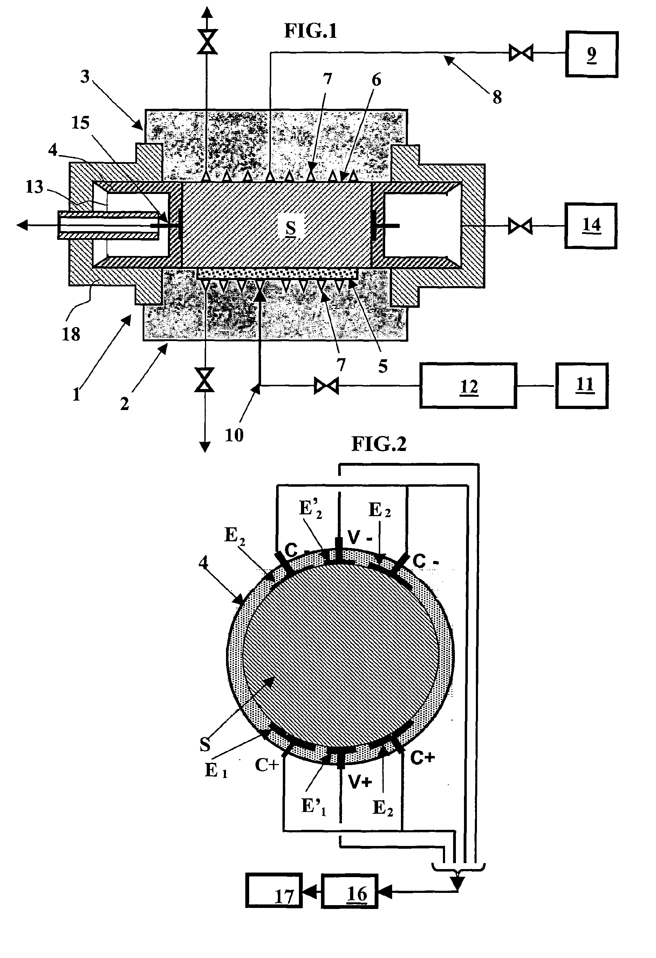

Il comporte par exemple (Fig.1) une cellule de confinement d'une carotte qui comporte un corps creux 1 à symétrie cylindrique fermé à ses deux extrémités opposées par deux embouts 2, 3. L'échantillon S est placé à l'intérieur d'une pièce cylindrique en élastomère 4 dont la section longitudinale est en forme de U, constituant une gaine pour l'échantillon S. L'ensemble de l'échantillon S et de sa gaine 4, est installé dans une cavité intérieure du corps 1 et se trouve délimité axialement de part et d'autre par les deux embouts 2, 3. Du côté de l'embout 2, l'échantillon S est en contact avec un filtre semi-perméable 5, mouillable par le premier fluide tel qu'un filtre en céramique. Du côté de l'embout 3 opposé, l'échantillon S est en contact avec une membrane 6 mouillable par le deuxième fluide. Les faces intérieures des deux embouts 2, 3 sont pourvues d'un réseau de rainures 7 (Fig.2). Des moyens de fixation non représentés permettent de fixer rigidement l'un à l'autre les deux embouts.

Des canaux 8 traversent l'embout 3 et font communiquer le réseau de rainures 7 sur sa face terminale, avec une première source 9 délivrant le deuxième fluide sous pression. De même, des canaux 10 traversent l'embout 2 et font communiquer le réseau de rainures 7 correspondant avec un circuit 11 de récupération du premier fluide drainé hors de l'échantillon du fait de l'injection du deuxième fluide. Un élément 12 est installé sur le circuit 9 pour mesurer le volume de fluide déplacé hors de l'échantillon S. On utilise de préférence un capteur capacitif de faible coût possédant une précision de 0,05 cc et d'une résolution de 0,01 cc, analogue à celui utilisé dans le dispositif décrit dans la demande de brevet FR 2.772.477 du demandeur.

Le dispositif comporte par exemple deux couples d'électrodes E1, E2 qui sont moulées à l'intérieur de la gaine 4, de façon à s'appliquer étroitement contre la paroi périphérique de l'échantillon, permettant l'application d'un courant électrique. Au moyen d'un autre couple d'électrodes E'1, E'2, pareillement moulées, on mesure la différence de potentiel V créée en réponse à l'application du courant électrique.

Cette affectation séparée des couples d'électrodes, les uns, à l'application d'un courant, et l'autre, à la mesure de différences de potentiel, permet d'éviter les résistances dues aux contacts. Les électrodes sont par exemple de forme carrée et réalisées en Monel. L'extension angulaire d'une paire d'électrodes autour de l'échantillon est inférieure à 90°. Leur longueur doit être inférieure à la longueur de l'échantillon de façon à éviter les courts-circuits électriques d'extrémité extérieurs à l'échantillon, directement au travers des fluides, ce qui fausseraient les mesures,. Cependant leur longueur doit être suffisamment importante rapportée à la longueur de l'échantillon de façon que les lignes de courant embrassent la plus grande partie de son volume avec une répartition relativement régulière. Cette longueur peut varier dans de notables proportions selon l'importance du diamètre de l'échantillon. Dans les expériences qui ont été réalisées, on a trouvé que la longueur des électrodes pouvait avantageusement être comprise entre ¼ et ¾ de la longueur de l'échantillon et de préférence être de l'ordre de la moitié de cette longueur.

L'espace annulaire 13 entre le corps 1 et la gaine 4 communique avec des moyens de pression 14 permettant l'injection d'un liquide sous pression qui exerce une pression radiale de confinement sur l'échantillon S. La pression radiale de confinement autour de l'échantillon est par exemple de l'ordre de quelques Mpa, suffisante pour assurer un bon contact électrique des électrodes. Ainsi, dans des conditions normales, la résistance de contact est généralement du même ordre de grandeur que la résistance de l'échantillon qui doit être mesurée avec une faible saturation en eau.

L'ensemble est placé dans une enceinte thermostatée (non représentée).

Toutes les électrodes P sont pourvues d'un prolongement creux 15 traversant l'épaisseur de la gaine 4, et sont reliées à un impédancemètre RLC 16 couplé avec un ensemble 17 d'acquisition de mesures.

MISE EN OEUVRE

L'échantillon S dont on cherche à mesurer l'anisotropie de résistivité est un barreau de roche (du grès par exemple) présente un litage telle qu'une fine couche d'argile. Cet échantillon est saturé avec un premier fluide.

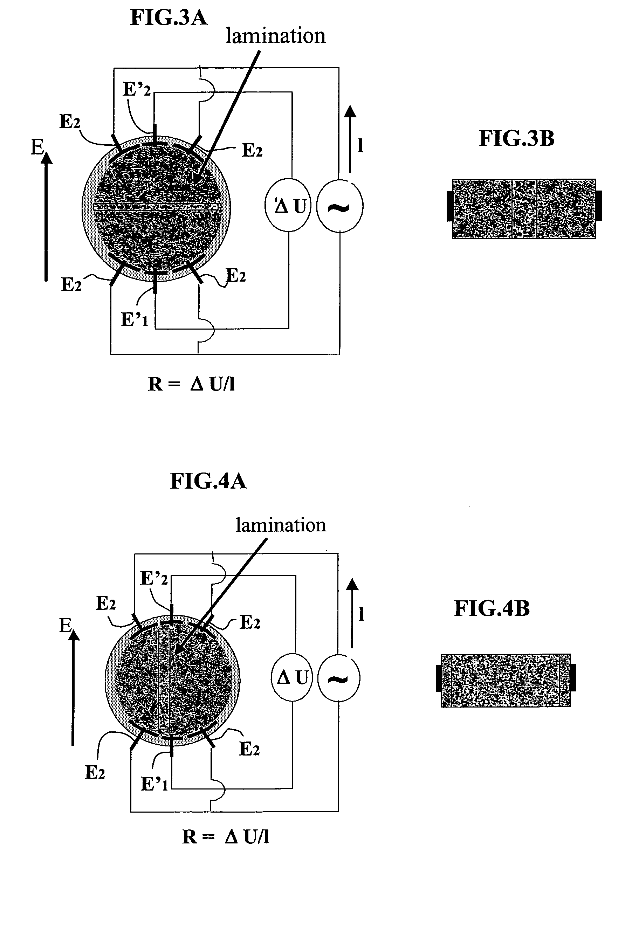

Dans un premier temps (Fig.3A, 3B), on le place dans la gaine 4 de façon que la direction d'allongement du litage soit sensiblement perpendiculaire au champ électrique EP que l'on va créer dans le barreau par l'application du courant entre les électrodes E2, et détecter par les électrodes E'1, E'2 et l'on applique une pression de confinement radiale par connexion avec les moyens de pression 14.

On injecte alors par les canaux 8 un deuxième fluide tel que de l'huile à une première pression et l'on mesure en continu les variations de l'impédance complexe de l'échantillon pour plusieurs fréquences entre 0.1 Hz et quelques dizaines de MHz., qui sont enregistrées par l'ensemble d'acquisition 16, 17. Les données sont analysées en utilisant un index de résistivité généralisé ou index d'impédance fonction de la saturation et de la fréquence f, défini comme suit :

$$Ir(Sw) = \frac{|Z(sw)|}{|Z(Sw = 1)|} = g(Sw, f)$$

où

$$|Z| = (Re(Z)^2 + Im(Z)^2)^{½}$$

Dans un deuxième temps, on répète les opérations précédentes mais après avoir fait tourner le barreau sur lui-même de façon que le litage soit maintenant parallèle à la direction du champ électrique EP (Fig.4A, 4B).

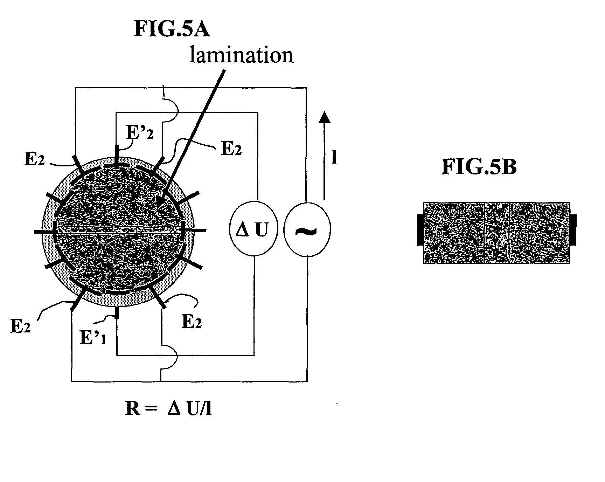

Pour éviter d'avoir à démonter la cellule de confinement pour atteindre le barreau et le faire tourner sur lui-même et changer son orientation par rapport au champ électrique, il est possible d'utiliser une cellule avec une répartition d'électrodes tout autour de la périphérie du barreau et de les connecter sélectivement à l'appareil de mesure (16, 17) de façon que le champ électrique EP qui se crée au travers du barreau une fois mis en place dans la gaine et application de courant électrique, soit alternativement perpendiculaire et parallèle au litage (Fig.5A, 5B).

**Exploitation des résultats**

A partir des mesures d'impédance qui ont été successivement faites dans les deux directions, on en déduit l'anisotropie de résistivité de l'échantillon.

Une méthode récente d'exploitation permet à partir de la connaissance de l'anisotropie de résistivité de remonter à la saturation dans les couches produisant des hydrocarbures. Des résultats d'anisotropie de résistivité pour l'interprétation des diagraphies de résistivité sont exposés dans « Anisotropy of resistivity in oil bearing thin-bedded formation : experiment and modeling, Clavaud J.B. and J. Lavigne, Proceedings of the 2003 International Conference of the Society of Core Analysts, 21-24 Pau, Septembre 2003.

La connaissance de la fraction volumique des laminations et de l'anisotropie de résistivité telle que mesurée dans le système décrit permet le calcul de la saturation en eau des couches pouvant produire des hydrocarbures.

**Revendications**

1. Méthode pour mesurer l'anisotropie de résistivité d'un échantillon poreux traversé par au moins un litage de conductivité différente, tel qu'une lamination, cet échantillon étant initialement saturé par un premier fluide, comportant la mise en place de l'échantillon dans un dispositif comprenant une cellule de confinement allongée (1), avec un premier filtre (M) semi-perméable, perméable au premier fluide et disposé sensiblement au contact d'une première extrémité de l'échantillon, et des moyens de (14) pour l'injection sous pression d'un deuxième fluide au travers d'une deuxième extrémité de l'échantillon, l'application contre l'échantillon d'électrodes (E) permettant l'application d'un courant électrique et la détection des différences de potentiel apparaissant entre des points d'application distincts en réponse à l'application du courant électrique, l'établissement d'au moins un palier de pression d'injection du deuxième fluide et la réalisation de mesures précises en continu des variations de l'impédance électrique complexe de l'échantillon à plusieurs fréquences durant une phase de déplacement du fluide saturant, **caractérisée en ce que** :

- a) on réalise des mesures de l'impédance présentée par l'échantillon dans une position où les litages sont orientées sensiblement transversalement au champ électrique (EF) créé par l'application du courant électrique ;

- b) on réalise des mesures de l'impédance présentée par l'échantillon dans une position où les litages sont orientées sensiblement dans la direction du champ électrique créé par l'application du courant électrique ; et

- c) on détermine l'anisotropie de résistivité.

2. Méthode selon la revendication 1, dans laquelle on change l'orientation de l'échantillon par rapport à la direction fixe du champ électrique créé par l'application du courant électrique, avant de procéder à l'étape b).

3. Méthode selon la revendication 1, dans laquelle on change la direction du champ électrique (EF) créé par l'application du courant électrique, avant de procéder à l'étape b).

4. Méthode selon l'une des revendications 1 à 3, **caractérisée en ce que** l'on utilise des électrodes dont la longueur est comprise entre ¼ et ¾ de la longueur de l'échantillon.

5. Méthode selon l'une des revendications 1 à 3, **caractérisée en ce que** l'on utilise des électrodes dont la longueur est de l'ordre de la moitié de la longueur de l'échantillon.

6. Méthode selon l'une des revendications précédentes, **caractérisée en ce que** l'on réalise des mesures précises en continu des variations de l'impédance électrique complexe de l'échantillon à plusieurs fréquences durant une phase de drainage.

7. Méthode selon l'une des revendications précédentes, **caractérisée en ce que** l'on réalise des mesures précises en continu des variations de l'impédance électrique complexe de l'échantillon à plusieurs fréquences durant une phase d'imbibition.

8. Dispositif pour mesurer l'anisotropie de résistivité d'un échantillon poreux (S) traversé par au moins un litage tel qu'une lamination de perméabilité différente, comprenant une cellule de confinement pour un échantillon (S) initialement saturé par un premier fluide, des paires d'électrodes (E) plaquées contre la périphérie de l'échantillon permettant l'application d'un courant électrique et la détection des différences de potentiel apparaissant entre des points distincts de l'échantillon (S) en réponse à l'application du courant électrique, les électrodes (E) étant connectées à un appareil (16, 17) de mesure de l'impédance de l'échantillon, un premier filtre semi-perméable (5), perméable au premier fluide et disposé sensiblement au contact d'une première extrémité de l'échantillon, et des moyens (8, 9) d'injection pour l'injection sous pression d'un deuxième fluide au travers d'une deuxième extrémité de l'échantillon, **caractérisé en ce qu'**il comporte une pluralité de couples d'électrodes (E) répartis sur le pourtour de l'échantillon qui peuvent être sélectivement connectées à l'appareil de mesure (16, 17) de façon à orienter le champ électrique (EF) créé par application de courant électrique à l'échantillon respectivement dans la même direction que le litage à l'étape b) et dans une direction transverse à l'étape a).

## FIG.1

## FIG.2

**FIG.3A**

lamination

E'₂

E₂

E

E₂

E₂

E₂

E'₁

I

ΔU  ~

**FIG.3B**

R = ΔU/l

**FIG.4A**

lamination

E'₂

E₂

E

E₂

E₂

E₂

E'₁

I

ΔU  ~

**FIG.4B**

R = ΔU/l

**FIG.5A**

lamination

$E'_2$

$E_2$

$E_2$

$E_2$

$E'_1$

$E_2$

$\Delta U$

$\sim$

$l$

$R = \Delta U/l$

**FIG.5B**